(19) **Europäisches Patentamt European Patent Office Office européen des brevets**

(11) **EP 4 703 466 A1**

(12) **EUROPEAN PATENT APPLICATION**
published in accordance with Art. 153(4) EPC

(43) Date of publication:
04.03.2026 Bulletin 2026/10

(21) Application number: 23935151.3

(22) Date of filing: 20.12.2023

(51) International Patent Classification (IPC):
$C12N\ 5/10^{(2006.01)}$    $C07K\ 19/00^{(2006.01)}$
$C12N\ 15/62^{(2006.01)}$    $A61K\ 39/00^{(2006.01)}$
$A61P\ 35/02^{(2006.01)}$

(86) International application number:
PCT/CN2023/140087

(87) International publication number:
WO 2024/221985 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.04.2023 CN 202310477837

(71) Applicant: Carbiogene Therapeutics Co., Ltd.
Hangzhou, Zhejiang 310051 (CN)

(72) Inventors:
• YANG, Xin
Hangzhou, Zhejiang 310051 (CN)
• ZHU, Jiangao
Hangzhou, Zhejiang 310051 (CN)
• YANG, Wenjun
Hangzhou, Zhejiang 310051 (CN)
• YU, Jiandong
Hangzhou, Zhejiang 310051 (CN)

(74) Representative: Winter, Brandl - Partnerschaft
mbB
Alois-Steinecker-Straße 22
85354 Freising (DE)

Remarks:
The complete document including Reference
Table(s) and the Sequence Listing(s) can be
downloaded from the EPO website

(54) **CAR-T CELL TARGETING CLL1, PREPARATION METHOD THEREFOR AND USE THEREOF**

(57) The present application relates to a CAR-T cell targeting CLL1, and preparation method and use thereof. Specifically, the present application relates to a CAR-T cell, containing a chimeric antigen receptor. The chimeric antigen receptor comprises a single-domain antibody, a hinge region, a transmembrane region, and an intracellular signaling region. The amino acid sequence of the single-domain antibody corresponds to positions 22-150 of SEQ ID No: 1. Experiments demonstrate that the CLL1-VHH-16 CAR-T cell is capable of effectively secret-ing T-cell-specific effector molecule IFN-γ, efficiently and specifically killing CLL1$^+$ target cells, and exhibiting excellent in vivo antitumor activity. The CLL1-VHH-16 CAR-T cell can not only significantly inhibit the proliferation of tumor cells in mice, but also significantly prolong the survival time of the mice. The CLL1-VHH-16 CAR-T cell exhibits excellent antitumor ability, can be used for immunotherapy of CLL1 target-related diseases, and has broad clinical application prospects.

EP 4 703 466 A1

## Description

### Technical Field

The present invention relates to the technical field of cellular immunotherapy, and more particularly to a CAR-T cell targeting CLL1, and the preparation method and use thereof.

### Background

Acute myeloid leukemia (AML) is a malignant disease of myeloid hematopoietic stem/progenitor cells. Anthracycline and cytarabine chemotherapy have long been used to treat low-risk patients, and hematopoietic stem cell transplantation has been used to treat intermediate and high-risk patients. The heterogeneity of acute myeloid leukemia makes the current treatment of AML patients still a challenge. In recent years, researchers have found that the immune targeted therapy targeting CLL1 is effective in treating AML. A C-type lectin-like molecule 1 (CLL1), also known as a C-type lectin domain family 12 member A (CLEC12A), is a type II transmembrane glycoprotein that plays an important role in immune regulation as an inhibitory receptor. CLL1 is present in myeloid cells of peripheral blood and bone marrow and most AML cells. It is also expressed on CD34$^+$CD38$^-$ stem cells of most AML, but not on CD34$^+$CD38$^-$ stem cells of normal people. CLL1 has become a potential target for AML treatment and diagnosis due to its special expression pattern. In addition, CLL1 is also expressed on cells of myelodysplastic syndromes (MDS) and chronic myeloid leukemia (CML).

Traditional radiotherapy, chemotherapy and hematopoietic stem cell transplantation have low efficacy in treating AML, but the emergence of CAR-T cell therapy has made it possible to cure AML. The full name of CAR-T is chimeric antigen receptor T-cell immunotherapy. A chimeric antigen receptor technology involves using a genetic engineering technology to modify immune cells, enabling them to express an exogenous antitumor gene (CAR gene), such that immune cells such as lymphocytes have the ability to recognize tumor cell surface antigens, and to specifically recognize and kill tumor cells without the restriction of a histocompatibility complex (MHC). The structure of CAR is mainly composed of an extracellular domain that recognizes a tumor cell surface antigen and an intracellular signal transduction domain. The extracellular domain is used to specifically recognize a tumor surface specific protein (antigen). The intracellular domain contains a co-stimulatory molecule domain, which is used to initiate the immune response of lymphocytes after recognizing the tumor surface specific protein (antigen), and exert cytotoxic effects on specific target cells, thereby killing target cells (tumor cells). At present, a CAR-T technology has been applied to clinical medicine and has achieved many successful clinical cases around the world. CAR-T cell therapy has achieved encouraging results in blood system tumors such as B cells, but the antigenic properties of the myeloid system are fundamentally different from those of the B system. The killing of normal myeloid cells by CAR-T will lead to bone marrow failure and a corresponding replacement therapy lacks, which has made the progress of CAR-T treatment of AML very slow. The successful application of the CAR-T technology in AML still faces many challenges. Therefore, further in-depth research and development of novel and effective CAR-T cells and improving the efficacy of CAR-T cells have important theoretical significance and clinical application value for immune cell therapy of AML.

### Summary of the Invention

One of the objects of the present invention is to provide a CAR-T cell targeting CLL1 for the treatment of acute myeloid leukemia (AML). The technical problem to be solved is not limited to the described technical schemes. Other technical themes which are not mentioned herein can be clearly understood by those skilled in the art through the following description.

To fulfill said objects, the present invention first provides a CAR-T cell. The CAR-T cell contains a chimeric antigen receptor, wherein the chimeric antigen receptor may include a single-domain antibody, a hinge region, a transmembrane region, and an intracellular signaling region; and an amino acid sequence of the single-domain antibody is positions 22-150 of SEQ ID No. 1.

The CAR-T cell (i.e., a T cell expressing CAR or a CAR-modified T cell) may be a CAR-T cell targeting CLL1, and the CAR-T cell expresses the chimeric antigen receptor.

Further, the intracellular signaling region may include a co-stimulatory domain 4-1BB and an activation domain CD3ζ, an amino acid sequence of the 4-1BB may be positions 220-266 of SEQ ID No. 1, and an amino acid sequence of the CD3ζ may be positions 267-378 of SEQ ID No. 1. Further, the hinge region may be a CD8a hinge region having an amino acid sequence as shown in positions 151-195 of SEQ ID No. 1, and the transmembrane region is a CD8a transmembrane region having an amino acid sequence as shown in positions 196-219 of SEQ ID No. 1.

Further, the chimeric antigen receptor may be named as CLL1-VHH-16 CAR, and may include any one of the followings:

A1) a protein having an amino acid sequence as shown in positions 22-378 of SEQ ID No. 1;

A2) a protein which is obtained by substitution and/or deletion and/or addition of an amino acid residue in the amino acid sequence as shown in positions 22-378 of SEQ ID No. 1, has the identity of 80% or more with the protein shown in A1) and has the same function as the protein shown in A1); and

A3) a fusion protein which is obtained by linking a tag or a signal peptide to an N-terminal and/or a C-terminal of A1) or A2) and has the same function as that of A1).

Further, an amino acid sequence of the signal peptide in A3) may be positions 1 to 21 of SEQ ID No. 1.

Further, the chimeric antigen receptor may include any one of the followings:

E1) a protein having an amino acid sequence as shown in SEQ ID No. 1;

E2) a protein which is obtained by substitution and/or deletion and/or addition of an amino acid residue in the amino acid sequence as shown in SEQ ID No, 1, has the identity of 80% or more with the protein shown in E1) and has the same function as the protein shown in E1); and E3) a fusion protein which is obtained by linking a tag to an N-terminal and/or a C-terminal of E1) or E2) and has the same function as that of E1).

The tag described herein includes, but is not limited to: a glutathione S-transferase (GST)-tag protein, a His-tag protein, a maltose-binding protein (MBP)-tag protein, a Flag-tag protein, a SUMO-tag protein, an HA-tag protein, an Myc-tag protein, an enhanced green fluorescent protein (eGFP), an enhanced cyan fluorescent protein (eCFP), an enhanced yellow-green fluorescent protein (eYFP), an mCherry (monomeric red fluorescent protein), or an AviTag-tag protein.

A person of ordinary skill in the art can readily mutate the nucleotide sequence that codes the chimeric antigen receptor of the present invention by using known methods, such as directed evolution or point mutation. Those artificially modified nucleotides that have the identity of 75% or more with the nucleotide sequence of the chimeric antigen receptor of the present invention, as long as they are capable of coding the chimeric antigen receptor and have a function of the chimeric antigen receptor, are all derived from the nucleotide sequences of the present invention and are equivalent to the sequences of the present invention.

Any chimeric antigen receptor herein is also within the protection scope of the present invention. The chimeric antigen receptor may include the signal peptide, the single-domain antibody, the hinge region, the transmembrane region, the co-stimulatory domain and the activation domain in sequence from the N-terminal to the C-terminal.

The present invention further provides a biomaterial. The biomaterial may be any one of the followings:

B1) a nucleic acid molecule that codes any chimeric antigen receptor described herein;

B2) an expression cassette containing the nucleic acid molecule of B1);

B3) a recombinant vector containing the nucleic acid molecule of B1), or a recombinant vector containing the expression cassette of B2); and

B4) a recombinant microorganism containing the nucleic acid molecule of B1), or a recombinant microorganism containing the expression cassette of B2), or a recombinant microorganism containing the recombinant vector of B3).

In the above biomaterial, the nucleic acid molecule of B1) may be any one of the followings:

C1) a DNA molecule having an amino acid sequence or a coding sequence as shown in positions 64-1134 of SEQ ID No. 2;

C2) a DNA molecule having the identity of 75% or more with the nucleotide sequence defined in C1) and having the same function as the DNA molecule of C1);

C3) a DNA molecule having a nucleotide sequence or a coding sequence as shown in SEQ ID No. 2; and

C4) a DNA molecule that has the identity of 75% or more with the nucleotide sequence defined in C3) and has the same function as the DNA molecule of C3).

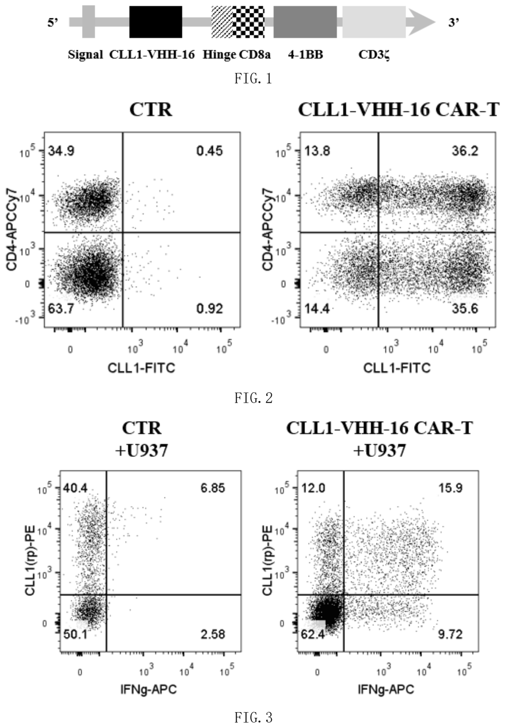

In an embodiment of the present invention, the chimeric antigen receptor is named as CLL1-VHH-16 CAR, and includes a signal peptide, a single-domain antibody (CLL1-VHH-16), a CD8a hinge region, a CD8a transmembrane region, a co-stimulatory domain 4-1BB and an activation domain CD3ζ in sequence from an N-terminal and a C-terminal (FIG. 1). The single-domain antibody is a single-domain antibody CLL1-VHH-16 that specifically binds to a CLL1 protein. The single-domain antibody CLL1-VHH-16 is an extracellular antigen binding region of the CAR, which is responsible for recognizing and binding to a tumor surface antigen CLL1. A section of signal peptide is carried in front (the N-terminal) of the antigen binding region, and used to guide a peptide chain of the antigen binding region to transfer to the outside of the cell, thereby recognizing the tumor cell surface antigen CLL1. The CD8a hinge region is a section of peptide chain of the CAR located outside the cell, and used to link the extracellular antigen binding region and the CD8a transmembrane region. The CD8a transmembrane region is used to anchor the single-domain antibody (CLL1-VHH-16) on a cell membrane. The intracellular signal region includes a co-stimulatory domain 4-1BB and an activation domain CD3ζ, which are used to synergistically stimulate a T cell and activate an intracellular signal, such that the T cell can continue to proliferate and release cytokines, improve the antitumor ability of the T cell, and at the same time exert a T cell signal transduction function to promote a stronger and more lasting T cell response.

An amino acid sequence of the CLL1-VHH-16 CAR is as shown in SEQ ID No. 1, wherein positions 1-21 of SEQ ID No. 1 are an amino acid sequence of the signal peptide; positions 22-150 of SEQ ID No. 1 are an amino acid sequence of the single-domain antibody (CLL1-VHH-16); positions 151-195 of SEQ ID No. 1 are an amino acid sequence of the CD8a hinge region; positions 196-219 of SEQ ID No. 1 are an amino acid sequence of the CD8a transmembrane region; positions 220-266 of SEQ ID No. 1 are an amino acid sequence of the co-stimulatory domain 4-1BB; and positions 267-378 of SEQ ID No. 1 are an amino acid sequence of the activation domain CD3ζ.

A coding gene of the CLL1-VHH-16 CAR is a CLL1-VHH-16 CAR gene, and has a nucleotide sequence as shown in SEQ ID No. 2, wherein positions 1-63 of SEQ ID No. 2 are a nucleotide sequence of the signal peptide; positions 64-450 of SEQ ID No. 2 are a nucleotide sequence of the single-domain antibody (CLL1-VHH-16); positions 451-585 of SEQ ID No. 2 are a nucleotide sequence of the CD8a hinge region; positions 586-657 of SEQ ID No. 2 are a nucleotide sequence of the CD8a transmembrane region; positions 658-798 of SEQ ID No. 2 are a nucleotide sequence of the co-stimulatory domain 4-1BB; and positions 799-1134 of SEQ ID No. 2 are a nucleotide sequence of the activation domain CD3ζ.

The above identity of 75% or more may be the identity of 80%, 85%, 90% or 95% or more.

The above identity of 80% or more may be the identity of at least 80%, 81%, 82%, 83%, 84%, 85%, 86%, 87%, 88%, 89%, 90%, 91%, 92%, 93%, 94%, 95%, 96%, 97%, 98% or 99%. The identity of 85% or more may be the identity of at least 85%, 86%, 87%, 88%, 89%, 90%, 91%, 92%, 93%, 94%, 95%, 96%, 97%, 98% or 99%. The identity of 90% or more may be the identity of at least 90%, 91%, 92%, 93%, 94%, 95%, 96%, 97%, 98% or 99%. The identity of 95% or more may be the identity of at least 95%, 96%, 97%, 98% or 99%.

As used herein, the identity refers to the identity of an amino acid sequence or nucleotide sequence. The identity of the amino acid sequence may be determined using a homology search site on Internet, such as a BLAST page of the NCBI homepage website. For example, in advanced BLAST2.1, with blastp as a program, an Expect value is set as 10, and all Filters are set as OFF; with BLOSUM62 as Matrix, the Gap existence cost, the Per residue gap cost, and the Lambda ratio are set as 11, 1 and 0.85 (default values), respectively; and the identity of the amino acid sequence is searched and calculated, and then the value of identity (%) can be obtained.

The vector as described herein refers to a vector that can transport exogenous DNA or a target gene into a host cell for amplification and expression. The vector may be a cloning vector or an expression vector, including but not limited to: a plasmid, phage (e.g., λ phage or M13 filamentous phage), clay (i.e., Cosmid), and a virus vector (e.g., baculovirus vector, retrovirus (including lentivirus), adenovirus, adeno-associated virus or herpes virus (e.g., herpes simplex virus)). In one or more embodiments of the present invention, the vector is a pUC57 vector and/or a retrovirus vector MP71.

The microorganism as described herein may be bacteria, yeast, algae or fungus. The bacteria may be derived from, but not limited to: *Escherichia sp., Erwinia sp., Agrobacterium sp., Flavobacterium sp., Alcaligenes sp., Pseudomonas sp., Bacillus sp.,* etc. The yeast may be derived from, but not limited to: *Saccharomyces sp., Pichia sp., Yarrowia sp., Candida sp., Schizosaccharomyces sp., Hansenula sp., Kluyveromyces sp.,* etc. The algae may be derived from, but not limited to: *Fucus sp., Achnanthes sp., Amphiprora sp., Amphora sp., Ankistrodesmus sp., Asteromonas sp., Boekelovia sp.,* etc. The fungus may be derived from, but not limited to: *Fusarium sp., Rhizoctonia sp., Verticillium sp., Penicillium sp., Aspergillus sp., Cephalosporium sp.,* etc. In one or more embodiments of the present invention, the microorganism is *Escherichia coli* DH5α.

The recombinant vector as described herein refers to a recombinant vector DNA molecule constructed by linking an exogenous target gene to a vector in vitro, and can be constructed in any suitable manner, as long as the constructed recombinant vector can carry the exogenous target gene into a receptor cell and provide the exogenous target gene with replication, integration, amplification and/or expression capabilities in the receptor cell. In one or more embodiments of the present invention, the recombinant vector is a recombinant retrovirus vector MP71-CLL1-VHH-16 CAR.

The recombinant retrovirus vector MP71-CLL1-VHH-16 CAR is a recombinant expression vector which is obtained by replacing fragments (small fragments) between *NotI* and *EcoRI* recognition sites of a retrovirus vector MP71 with a DNA fragment as shown in SEQ ID No. 2 in a sequence listing, and keeping the other nucleotide sequences of the retrovirus vector MP71 unchanged.

The recombinant microorganism as described herein refers to a microorganism with a changed function obtained by operating and modifying genes of a target microorganism, such as a recombinant microorganism obtained by introducing an exogenous target gene or a recombinant vector into a target microorganism, or a recombinant microorganism obtained by directly editing endogenous genes of a target microorganism. In one or more embodiments of the present invention, the recombinant microorganism is recombinant bacteria obtained by introducing the recombinant retrovirus vector MP71-CLL1-VHH-16 CAR into *Escherichia coli* DH5α, which contain the DNA molecule as shown in SEQ ID No. 2.

The CAR-T cell as described herein is a recombinant cell obtained by transferring the constructed chimeric antigen receptor (CAR) gene into a T cell for stable expression by means of a genetic engineering in vitro recombination method. Further, the CAR-T cell as described herein may be a CLL1-VHH-16 CAR-T cell obtained by transferring the CLL1-VHH-16 CAR gene (SEQ ID No. 2) into the T cell for stable expression.

Specifically, the CLL1-VHH-16 CAR-T cell may be a recombinant cell obtained by packaging a recombinant retrovirus vector MP71-CLL1-VHH-16 CAR through packaging cells to obtain a recombinant retrovirus, and then introducing the recombinant retrovirus into the T cell. The packaging cells may be Phoenix Ecotropic (ECO) cells and PG13 cells.

The CLL1-VHH-16 CAR-T cell contains and/or expresses the CLL1-VHH-16 CAR gene (SEQ ID No. 2).

The CLL1-VHH-16 CAR-T cell as described herein has at least any one of the following characteristics:

F1) secreting a specific effector molecule IFN-y;
F2) specifically killing a CLL1$^+$ tumor cell; and
F3) inhibiting the proliferation of the CLL1$^+$ tumor cell.

The present invention further provides a use of the chimeric antigen receptor CLL1-VHH-16 CAR in the preparation of a CLL1-VHH-16 CAR-T cell.

The present invention further provides a pharmaceutical composition, which may contain any of the CAR-T cells as described herein.

The pharmaceutical composition is used for preventing or treating a CLL1 target-related disease. Further, the pharmaceutical composition further includes one or more pharmaceutically acceptable vectors.

Further, the pharmaceutically acceptable vector may be an excipient, a stabilizer, a suspending agent or a diluent, etc., which are well known to a person skilled in the art.

The present invention further provides a use of any of the CAR-T cells as described herein, or the chimeric antigen receptor, or the biomaterial, or the pharmaceutical composition, including any one of the followings:

D1) a use in the preparation of a drug for preventing or treating a tumor, or a use in the prevention or treatment of a tumor; and
D2) a use in the preparation of a drug for preventing or treating a CLL1 target-related disease, or a use in the prevention or treatment of a CLL1 target-related disease.

Further, in the above use, the CLL1 target-related disease may be CLL1-positive cancer (i.e., cancer expressing CLL1).

Further, the CLL1-positive cancer may be acute myeloid leukemia (AML), myelodysplastic syndrome (MDS) or chronic myeloid leukemia (CML).

The present invention further provides a preparation method for any of the CAR-T cells as described herein. The method may include: transferring any of the nucleic acid molecules as described herein into a T cell for stable expression to obtain the CAR-T cell.

Further, the method may include the following steps:

(1) constructing a CLL1-VHH-16 CAR gene (SEQ ID No. 2) between *NotI* and *EcoRI* recognition sites of a retrovirus vector MP71 to obtain a recombinant retrovirus vector MP71-CLL1-VHH-16 CAR;
(2) introducing the recombinant retrovirus vector MP71-CLL1-VHH-16 CAR into a packaging cell for packaging to obtain a recombinant retrovirus; and
(3) infecting a human T cell with the recombinant retrovirus to obtain a recombinant cell, namely a CLL1-VHH-16 CAR-T cell.

Further, the packaging cell may be a Phoenix Ecotropic (ECO) cell and a PG13 cell.

The present invention further provides a method for the prevention or treatment of a CLL1 target-related disease. The method may include: administering any of the CAR-T cells as described herein or the pharmaceutical composition to a subject suffering from the CLL1 target-related disease.

Further, the CLL1 target-related disease may be CLL1-positive cancer.

Further, the CLL1-positive cancer is acute myeloid leukemia, myelodysplastic syndrome or chronic myeloid leukemia.

The CLL1 A C-type lectin-like molecule 1 as described herein, also known as a C-type lectin domain family 12 member A (CLEC12A), is a type II transmembrane glycoprotein that plays an important role in immune regulation as an inhibitory receptor. CLL1 is present in myeloid cells of peripheral blood and bone marrow and most acute myeloid leukemia (AML) cells. It is also expressed on CD34$^+$CD38$^-$ stem cells of most AML, but not on CD34$^+$CD38$^-$ stem cells of normal people. CLL1 has become a potential target for AML treatment and diagnosis due to its special expression pattern.

The present invention designs a CAR-T cell (CLL1-VHH-16 CAR-T cell) targeting CLL1 with a novel structure. Experiments demonstrate that the CLL1-VHH-16 CAR-T cell of the present invention is capable of effectively secreting a T-cell-specific effector molecule IFN-γ, efficiently and specifically killing CLL1$^+$ target cells, and exhibiting good in vivo antitumor activity. The CLL1-VHH-16 CAR-T cell can not only significantly inhibit the proliferation of tumor cells in mice, but also significantly prolong the survival time of the mice. The CLL1-VHH-16 CAR-T cell of the present invention exhibits excellent antitumor ability, can be used for immunotherapy of CLL1 target-related diseases (acute myeloid leukemia, myelodysplastic syndrome or chronic myeloid leukemia), and has broad clinical application prospects.

**Brief Description of the Drawings**

FIG. 1 is a schematic structural diagram of a CLL1-VHH-16 CAR molecule.
FIG. 2 shows an assay for CAR expression in a CLL1-VHH-16 CAR-T cell.
FIG. 3 shows an assay for a cell function of a CLL1-VHH-16 CAR-T cell to secret IFN-γ.
FIG. 4 shows a cytotoxicity assay of the CLL1-VHH-16 CAR-T cell.
FIG. 5 shows an in vivo tumor-killing activity assay for the CLL1-VHH-16 CAR-T cell.

**Detailed Description of the Invention**

The present invention is described in further detail below in conjunction with the specific embodiments, and the given examples are only for the purpose of clarifying the present invention, but not for the purpose of limiting the scope of the present invention. The examples provided below may be used as a guide for further improvement by a person of ordinary skill in the art and do not in any way constitute a limitation of the present invention.

The experimental methods in the following examples, unless otherwise specified, are conventional methods and are carried out in accordance with the techniques or conditions described in the literatures in the art or in accordance with the product instructions. The materials, reagents, etc. used in the following examples, unless otherwise specified, may be obtained commercially.

A retrovirus vector MP71 in the following examples was described in the following document: Engels B, Cam H, et al. Retroviral Vectors for High-Level Transgene Expression in T Lymphocytes [J]. Human Gene Therapy, 2003, 14 (12): 1155-1168. The public can obtain this biomaterial from the applicant. This biomaterial is only used for repeating the experiments in the present invention and cannot be used for other purposes.

Human peripheral blood mononuclear cells (PBMCs) in the following examples were derived from the venous blood of healthy volunteers.

U937 cells (CLL1-positive cells) in the following examples were acute myeloid leukemia cells, a product from Beijing Biobw Biotech Co., Ltd., Catalog number: bio-73180.

**Example 1: Structure and sequence of chimeric antigen receptor (CAR)**

The chimeric antigen receptor designed and constructed in this example is named as CLL1-VHH-16 CAR, and includes a signal peptide, a single-domain antibody (CLL1-VHH-16), a CD8a hinge region, a CD8a transmembrane region, a co-stimulatory domain 4-1BB and an activation domain CD3ζ in sequence from an N-terminal to a C-terminal (FIG. 1).

The single-domain antibody is a single-domain antibody CLL1-VHH-16 that specifically binds to a CLL1 protein. The single-domain antibody CLL1-VHH-16 is an extracellular antigen binding region of the CAR, which is responsible for recognizing and binding to a tumor surface antigen CLL1. A section of signal peptide is carried in front (the N-terminal) of the antigen binding region, and used to guide a peptide chain of the antigen binding region to transfer to the outside of the cell, thereby recognizing the tumor cell surface antigen CLL1. The CD8a hinge region is a section of peptide chain of the CAR located outside the cell, and used to link the extracellular antigen binding region and the CD8a transmembrane region. The CD8a transmembrane region is used to anchor the single-domain antibody (CLL1-VHH-16) on a cell membrane. The intracellular signal region includes a co-stimulatory domain 4-1BB and an activation domain CD3ζ, which are used to synergistically stimulate a T cell and activate an intracellular signal, such that the T cell can continue to proliferate and release cytokines, improve the antitumor ability of the T cell, and at the same time exert a T cell signal transduction function to promote a stronger and more lasting T cell response.

An amino acid sequence of the CLL1-VHH-16 CAR as designed above is as shown in SEQ ID No. 1, wherein positions 1-21 of SEQ ID No. 1 are an amino acid sequence of the signal peptide; positions 22-150 of SEQ ID No. 1 are an amino acid sequence of the single-domain antibody (CLL1-VHH-16); positions 151-195 of SEQ ID No. 1 are an amino acid sequence of the CD8a hinge region; positions 196-219 of SEQ ID No. 1 are an amino acid sequence of the CD8a transmembrane region; positions 220-266 of SEQ ID No. 1 are an amino acid sequence of the co-stimulatory domain 4-1BB; and positions 267-378 of SEQ ID No. 1 are an amino acid sequence of the activation domain CD3ζ.

A coding gene of the CLL1-VHH-16 CAR is a CLL1-VHH-16 CAR gene, and has a nucleotide sequence as shown in SEQ ID No. 2, wherein positions 1-63 of SEQ ID No. 2 are a nucleotide sequence of the signal peptide; positions 64-450 of SEQ ID No. 2 are a nucleotide sequence of the single-domain antibody (CLL1-VHH-16); positions 451-585 of SEQ ID No. 2 are a nucleotide sequence of the CD8a hinge region; positions 586-657 of SEQ ID No. 2 are a nucleotide sequence of the CD8a transmembrane region; positions 658-798 of SEQ ID No. 2 are a nucleotide sequence of the co-stimulatory domain 4-1BB; and positions 799-1134 of SEQ ID No. 2 are a nucleotide sequence of the activation domain CD3ζ.

**Example 2: Preparation of CLL1-VHH-16 CAR-T cell**

The CAR-T cell prepared in this example was obtained by transferring the CLL1-VHH-16 CAR gene (SEQ ID No. 2) in Example 1 into a T cell for stable expression, thereby obtaining a T cell expressing the CLL1-VHH-16 CAR gene, which was named as a CLL1-VHH-16 CAR-T cell. The specific steps were as follows.

2-1. Construction of recombinant retrovirus vector

(1) A recombinant vector pUC57-CLL1-VHH-16 CAR was double-digested with *Not*I (NEB) and *Eco*RI (NEB), and target gene fragments were obtained by gel extraction. The recombinant vector pUC57-CLL1-VHH-16 CAR was obtained by cloning the CLL1-VHH-16 CAR gene (SEQ ID No. 2) into a pUC57 vector, and was synthesized and provided by Tsingke Biotech Co., Ltd.

(2) A retrovirus vector MP71 was double-digested with *Not*I and *Eco*RI, and large vector fragments were obtained by gel extraction.

(3) The above-mentioned target gene fragments and large vector fragments were linked through a T4 ligase (NEB) to obtain a recombinant retrovirus vector MP71-CLL1-VHH-16 CAR carrying the CLL1-VHH-16 CAR gene.

(4) The recombinant retrovirus vector MP71-CLL1-VHH-16 CAR was transformed into competent *Escherichia coli* DH5α, and plasmids were extracted and purified using a plasmid purification kit (Qiagen) to obtain MP71-CLL1-VHH-16 CAR plasmids.

The recombinant retrovirus vector MP71-CLL1-VHH-16 CAR (i.e., MP71-CLL1-VHH-16 CAR plasmids) was a recombinant expression vector which was obtained by replacing fragments (small fragments) between *Not*I and *Eco*RI recognition sites of the retrovirus vector MP71 with a DNA fragment as shown in SEQ ID No. 2 in a sequence listing, and keeping the other nucleotide sequences of the retrovirus vector MP71 unchanged.

2-2. Retrovirus packaging

The MP71-CLL1-VHH-16 CAR plasmids prepared in step 2-1 were introduced into packaging cells for packaging to complete virus assembly and obtain a retrovirus. The specific steps of virus packaging were as follows.

a) Day 1: Phoenix Ecotropic (ECO) cells should be less than 20 generations and not overgrown. The cells were plated at a density of $0.6 \times 10^6$ cells/ml, 10 mL of DMEM medium was added to a 10 cm dish, and the cells were mixed well and cultured at 37°C overnight.

b) Day 2: ECO cells were transfected (usually about 14-18 h after plating) when the confluence reached about 90%; 12.5 μg of plasmids, 250 μL of 1.25M CaCl$_2$, and 1 mL of H$_2$O were prepared in a total volume of 1.25 mL; and 2×HBS of the same volume as the plasmid complex was added to another tube, and vortexed and shaken for 20 s while adding the plasmid complex. The mixture was gently added to an ECO dish along the edge, cultured at 37°C for 4 h, washed once with PBS after a medium was removed, and added with a preheated fresh medium again.

c) Day 4: after 48 h of transfection, a supernatant was collected, and filtered with a 0.45 μm filter to obtain a retrovirus solution, which was then aliquoted and stored at -80°C.

d) 1.2 mL of 15 ug/mL RetroNectin coating solution was added to each well of a 6-well NTC plate and cultured at 4°C overnight.

e) a blocking solution was pipetted away carefully, PBS was added at 2 mL/well for washing, 5 mL of the above virus solution was added to each well and centrifuged at 32°C, 2000×g for 2 h, and the unbound virus supernatant was pipetted away.

f) PG13 cells in the logarithmic phase were taken and rinsed once with 10 mL of PBS, added with 1 mL of 0.25% recombinant trypsin, and allowed to stand at room temperature for 2-3 min.

g) 5 ml of complete medium containing 10% FBS was added to terminate digestion, and centrifuged at 1500 rpm for 5 min.

h) A supernatant was discarded, the cell density was adjusted to $0.5 \times 10^5$ cells/mL with the complete medium, and the cells were added to the above virus-coated 6-well NTC plate at 3 mL/well, such that the final cell count was $1.5 \times 10^5$ cells/well.

i) The cells were centrifuged at 1000 rpm for 1 min, and cultured at 37°C, 5% $CO_2$ for 48 h.

j) After 1-2 generations of subculture, the cells were transferred to a T175 culture flask and cultured in a DEME medium containing 12% FBS for 2 d.

k) After the cells were continuously cultured for 48 h in a new DEME medium containing 12% FBS, a supernatant was collected, and filtered with a 0.45 $\mu$m filter to obtain a retrovirus solution, which was then aliquoted and stored at -80°C.

2-3. Retrovirus-infected human T cells

a) Freeze-stored healthy human peripheral blood PBMC were thawed, and the cell density was adjusted to $1 \times 10^6$ to $2 \times 10^6$ cells/mL using an RPMI-1640 medium containing 10% fetal bovine serum (FBS).

b) PBMC was collected with a Ficoll separation solution (Tianjin Haoyang), CD3$^+$ T cells were separated by a magnetic bead method, and clinical-grade Dynabeads® Human T-Expander CD3/CD28 magnetic beads (Invitrogen) were added at a ratio of magnetic beads: CD3$^+$ cells=3: 1 to activate T cells.

c) On the second day after T cell activation, RetroNectin (TAKARA) diluted with PBS to a final concentration of 15 $\mu$g/mL was used to coat a non-tissue-treated culture plate (6-well plate) at 1.2 mL/well. The cells were cultured in the dark at 4°C overnight for later use.

d) After two days of T cell activation culture, the coated 6-well plate was taken out, a coating solution was pipetted away, and PBS was added to wash the plate once.

e) 5-6 mL of retrovirus solution prepared in step 2-2 was added to each well, and centrifuged at 32°C, 2000×g for 2 h. 3 mL of fresh complete medium containing hIL-2 (500 U/mL) was added to each well, and continued to culture for 1 d.

f) After cell infection, the cell density was observed every day, and a T cell culture solution containing 100 U/mL IL-2 was supplemented in time, such that the T cell density was maintained at about $5 \times 10^5$ cells/mL to facilitate cell expansion.

g) Thus, CAR-T cells infected with the retrovirus prepared in step 2-2 were obtained and named as CLL1-VHH-16 CAR-T cells (i.e., T cells expressing the CLL1-VHH-16 CAR gene having a nucleotide sequence as shown in SEQ ID No. 2).

The constructed CLL1-VHH-16 CAR-T cells and CTR T cells (i.e., T cells without virus transfection, as a control) were cultured in an RPMI-1640 medium containing 10% fetal bovine serum (FBS) at 37°C and recorded as Day D0. Various functional tests were performed until D10.

**Example 3. Assay for CAR expression in CLL1-VHH-16 CAR-T cell**

The constructed CLL1-VHH-16 CAR-T cell contained a CLL1-VHH-16 CAR gene having a nucleotide sequence as shown in SEQ ID No. 2, and expressed a chimeric antigen receptor CLL1-VHH-16 CAR having an amino acid sequence as shown in SEQ ID No. 1. The steps for assay for CAR expression were as follows.

1. After cell centrifugation (1500 rpm × 5 min), a supernatant was discarded, 200 $\mu$L of FACS buffer (1×PBS containing 0.1% NaN$_3$ and 2% FBS) was added to each well in a 96-well round-bottom plate, resuspended, and centrifuged at 1500 rpm for 5 min.

2. 60 $\mu$L of prepared fluorescently-labeled anti-human CD4-APC Cy7/CLL1-FITC was added to each well, resuspended and mixed well, and incubated at 4°C for 30 min.

3. 200 $\mu$L FACS buffer was added to each well, and centrifuged at 1500 rpm for 5 min.

4. A supernatant was discarded, the cells were resuspended in 400 $\mu$L of FACS buffer and transferred to a flow cytometry tube, and the cells were read using a flow cytometer (BD Canto-II) to analyze a percentage of a CLL1 antibody in the CLL1-VHH-16 CAR-T cell.

The assay results were shown in FIG. 2. The CLL1-VHH-16 CAR-T cell can well express the CLL1 antibody (i.e., a single-domain antibody CLL1-VHH-16), indicating that the expression of a CAR molecule in the CLL1-VHH-16 CAR-T cell met an expected design.

**Example 4. Assay for function of CLL1-VHH-16 CAR-T cell to secret specific effector molecule IFN-y**

1. The cell density of test cells, i.e., CLL1-VHH-16 CAR-T cells and CTR T cells (i.e., T cells without virus transfection, as a control), was adjusted to $2\times10^6$ cells/mL. 100 μL of cells were taken and added to a 96-well U-bottom plate. U937 cells were added at a ratio of test cells: target cells = 1:1. Brefeldin A (Med Chem Express, HY-16592) at a final concentration of 5 μg/mL was added to each well. The cells were incubated in a 37°C incubator for 6 h.

CTR+U937: 1 mL of $1\times10^6$ U937 cells in total were added into 1 mL of $1\times10^6$ non-virus-transfected T cells (CTR T cells) in total per well.

CLL1-VHH-16 CAR-T+U937: 1 mL of $1\times10^6$ U937 cells in total were added into 1 mL of $1\times10^6$ CLL1-VHH-16 CAR-T cells in total per well.

2. After incubation, flow cytometry staining was carried out. The operation steps were as follows:

(1) after cell centrifugation (1500 rpm × 5 min), a supernatant was discarded, 200 μL of FACS buffer (1×PBS containing 0.1% $NaN_3$ and 2% FBS) was added to each well, resuspended, and centrifuged at 1500 rpm for 5 min, and this step was repeated twice;

(2) 60 μL of prepared fluorescently-labeled CLL1(rp)-PE was added to each well, resuspended and mixed well, and incubated at room temperature for 10 min;

(3) 200 μL of FACS buffer was added to each well and centrifuged at 1500 rpm for 5 min, and a supernatant was then discarded;

(4) 150 μL of Cytofix/Cytoperm (BD, Catalog number: 55472) was added to each well, resuspended and mixed well, and incubated at room temperature in the dark for 15 min;

(5) a supernatant was discarded by centrifugation at 1500 rpm for 5 min, and 200 μL of Perm/Wash buffer (BD, Catalog number. 554723) was added to each well, resuspended and mixed well, centrifuged at 1500 rpm for 5 min, and centrifugally washed twice;

(6) 20 μL of diluted APC-labeled anti-human IFN-γ (BioLegend, Catalog number: 506510) was added to each well, resuspended and mixed well, and incubated at room temperature in the dark for 20 min; and

(7) 200 μL of Perm buffer was added to each well, and centrifuged at 1500 rpm for 5 min. After a supernatant was discarded, the cells were resuspended in 400 μL of FACS buffer and transferred to a flow cytometry tube, and the cells were read using a flow cytometer (BD Canto-II) to analyze the percentages of a functional effector molecule IFN-γ in the CLL1-VHH-16 CAR-T cell and the control cell.

The assay results were shown in FIG. 3. After co-culture with U937 target cells, the CLL1-VHH-16 CAR-T cells were able to secrete the T-cell-specific effector molecule IFN-γ well.

**Example 5. Cytotoxicity assay of CLL1-VHH-16 CAR-T cell**

1. The cell density of effector cells, i.e., CLL1-VHH-16 CAR-T cells and CTR T cells (i.e., T cells without virus transfection, as a control), was adjusted to $2\times10^5$ cells/mL. 100 μL of cells were taken and added to a 96-well U-bottom plate. D-firefly luciferin sodium salt (Yeasen Biotechnology, Catalog number: 40901ES08, having a storage concentration of 100 mg/mL) having a final concentration of 100 μg/mL was added, and duplicated in 3 wells.

2. Target cells U937-luc (the cell density of target cells under a condition of 1: 1 was $2\times10^4$ cells) according to different effector to target ratios (9:1, 3:1, 1:1, and 1:3) and cultured at 37°C for 16 h.

3. A fluorescence value was measured by using a TECAN spark microplate reader, and an average value of three replicate wells was taken to calculate the specific cytotoxicity of the CLL1-VHH-16 CAR-T cells:

$$\text{Specific lysis \%} = 100 - 100 \times (E_{exp} - E_{min})/(T_{max} - T_{min})$$

$E_{exp}$: RLU value when effector cells and target cells were co-cultured;

$E_{min}$: RLU value of spontaneous death of effector cells in the absence of cells;

$T_{max}$: RLU value of spontaneous death of target cells in the absence of effector cells; and

$T_{min}$: RLU value under a condition of maximum killing rate.

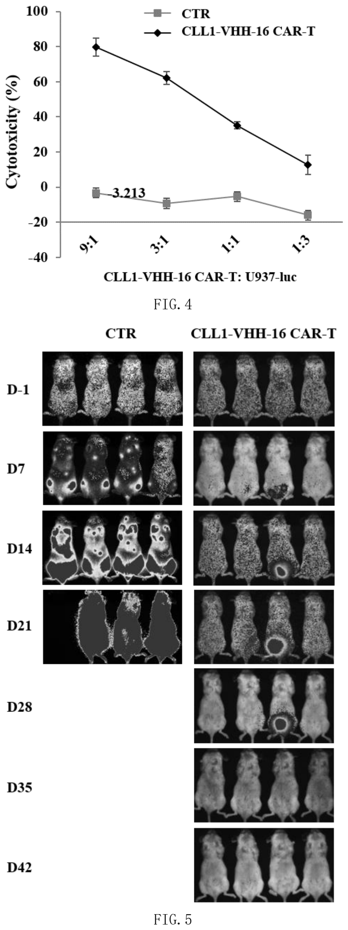

The assay results were shown in FIG. 4. The CLL1-VHH-16 CAR-T cells can effectively and specifically kill CLL1[+]

target cells (U937 cells), while the control CTR T cells had no killing effect on CLL1⁺ target cells.

**Example 6. In vivo tumor-killing activity assay for CLL1-VHH-16 CAR-T cells**

1. 8 female NSG mice (Shanghai Model Organisms Center, Inc., Catalog number: NM-NSG-001, 8-10 weeks old) each was inoculated with $2.0 \times 10^6$ U937-Luc-GFP cells via the tail vein.
2. After six days of inoculation, the mice were randomly divided into two groups, and each mouse was injected with $4.0 \times 10^6$ CLL1-VHH-16 CAR-T or CTR T (i.e., T cells without virus transfection, as a control) cells via the tail vein, and recorded as Day D0.
3. On D7, D14, D21, D28, D35, and D42, a Photo Acquisition small animal imager was used to capture tumor luminescence signals.

The assay results were shown in Table 1 and FIG. 5. Compared with the CTR control group, the CLL1-VHH-16 CAR-T cells can not only significantly inhibit the proliferation of tumor cells in the mice, but also significantly prolong the survival time of the mice. By D35 and D42, the tumor tissues in the mice had completely disappeared.

Table 1. Bioluminescence intensity (P/S) of tumor cells in tumor-bearing mice

| Groups | Serial Nos. | D-1 | D7 | D14 | D21 | D28 | D34 | D42 |
|---|---|---|---|---|---|---|---|---|
| CTR | 1 | 3.57E+06 | 7.29E+07 | 2.41E+09 | N/A | N/A | N/A | N/A |
| | 2 | 2.42E+06 | 4.77E+07 | 1.01E+09 | 6.39E+10 | N/A | N/A | N/A |
| | 3 | 2.87E+06 | 4.86E+07 | 2.41E+09 | 8.71E+09 | N/A | N/A | N/A |
| | 4 | 3.16E+06 | 1.04E+07 | 1.21E+09 | 3.42E+10 | N/A | N/A | N/A |
| CLL1-VHH-16 CAR-T | 1 | 1.34E+07 | 6.30E+05 | 7.73E+06 | 1.47E+07 | 8.19E+06 | 1.66E+06 | 5.84E+05 |
| | 2 | 1.58E+07 | 8.03E+05 | 4.86E+07 | 1.10E+09 | 2.27E+09 | 3.06E+06 | 7.71E+05 |
| | 3 | 1.50E+07 | 2.09E+06 | 8.03E+06 | 2.19E+07 | 1.19E+07 | 1.62E+06 | 4.25E+05 |
| | 4 | 1.31E+07 | 6.74E+05 | 7.22E+06 | 1.28E+07 | 3.05E+06 | 1.41E+06 | 4.46E+05 |

The present invention is detailed above. For a person skilled in the art, the present invention may be implemented within a wide range under the same parameters, concentrations and conditions without departing from the purpose and scope of the present invention and without unnecessary experiments. Although special examples are given in the present invention, it should be understood that further improvements may be made to the present invention. In summary, according to the principles of the present invention, the present application is intended to include any change, use or improvement of the present invention, including changes that deviate from the scope disclosed in the present application and are made by conventional technologies known in the art. According to the scope of the claims attached below, some basic features can be applied.

**Industrial Applicability**

The present invention designs a CAR-T cell (CLL1-VHH-16 CAR-T cell) targeting CLL1 with a novel structure. The CAR-T cell is capable of effectively secreting a T-cell-specific effector molecule IFN-γ, efficiently and specifically killing CLL1⁺ target cells, and exhibiting good in vivo antitumor activity. The CLL1-VHH-16 CAR-T cell can not only significantly inhibit the proliferation of tumor cells in mice, but also significantly prolong the survival time of the mice. The CLL1-VHH-16 CAR-T cell of the present invention exhibits excellent antitumor ability, can be used for immunotherapy of CLL1 target-related diseases (acute myeloid leukemia, myelodysplastic syndrome or chronic myeloid leukemia), and has broad clinical application prospects.

**Claims**

1. A CAR-T cell, containing a chimeric antigen receptor, wherein the chimeric antigen receptor comprises a single-domain antibody, a hinge region, a transmembrane region, and an intracellular signaling region; and the single-domain antibody consists of an amino acid sequence shown in positions 22-150 of SEQ ID No. 1.

2. The CAR-T cell according to claim 1, wherein the intracellular signaling region comprises a co-stimulatory domain 4-1BB and an activation domain CD3ζ, wherein the 4-1BB consists of an amino acid sequence shown in positions 220-266 of SEQ ID No. 1, and the CD3ζ consists of an amino acid sequence shown in positions 267-378 of SEQ ID No. 1.

3. The CAR-T cell according to claim 1 or 2, wherein the hinge region is a CD8a hinge region having an amino acid sequence as shown in positions 151-195 of SEQ ID No. 1, and the transmembrane region is a CD8a transmembrane region having an amino acid sequence as shown in positions 196-219 of SEQ ID No. 1.

4. The CAR-T cell according to any one of claims 1 to 3, wherein the chimeric antigen receptor comprises any one of the following:

   A1) a protein having an amino acid sequence as shown in positions 22-378 of SEQ ID No. 1;
   A2) a protein which is obtained by substitution and/or deletion and/or addition of an amino acid residue in the amino acid sequence as shown in positions 22-378 of SEQ ID No. 1, has the identity of 80% or more with the protein shown in A1) and has the same function as the protein shown in A1); and
   A3) a fusion protein which is obtained by linking a tag or a signal peptide to an N-terminal and/or a C-terminal of A1) or A2) and has the same function as that of A1).

5. The CAR-T cell according to claim 4, wherein the signal peptide in A3) consists of an amino acid sequence shown in positions 1-21 of SEQ ID No. 1.

6. A chimeric antigen receptor according to any one of claims 1 to 5.

7. A biomaterial, which is any one of the following:

   B1) a nucleic acid molecule encoding the chimeric antigen receptor according to claim 6;
   B2) an expression cassette containing the nucleic acid molecule of B1);
   B3) a recombinant vector containing the nucleic acid molecule of B1), or a recombinant vector containing the expression cassette of B2); and
   B4) a recombinant microorganism containing the nucleic acid molecule of B1), or a recombinant microorganism containing the expression cassette of B2), or a recombinant microorganism containing the recombinant vector of B3).

8. The biomaterial according to claim 7, wherein the nucleic acid molecule of B1) is any one of the following:

   C1) a DNA molecule having an amino acid sequence or a coding sequence as shown in positions 64-1134 of SEQ ID No. 2;
   C2) a DNA molecule having 75% or higher identity with the nucleotide sequence defined in C1) and having the same function as the DNA molecule of C1);
   C3) a DNA molecule having a nucleotide sequence or a coding sequence of SEQ ID No. 2; and
   C4) a DNA molecule having 75% or higher identity with the nucleotide sequence defined in C3) and has the same function as the DNA molecule of C3).

9. A pharmaceutical composition, comprising the CAR-T cell according to any one of claims 1 to 5.

10. Use of the CAR-T cell according to any one of claims 1 to 5, or the chimeric antigen receptor according to claim 6, or the biomaterial according to claim 7 or 8, or the pharmaceutical composition according to claim 9, in any one of the following:

    D1) use in the preparation of a drug for preventing or treating a tumor, or use in the prevention or treatment of a tumor; and
    D2) use in the preparation of a drug for preventing or treating a CLL1 target-related disease, or use in the prevention or treatment of a CLL1 target-related disease.

11. The use according to claim 10, wherein the CLL1 target-related disease is CLL1-positive cancer.

12. The use according to claim 11, wherein the CLL1-positive cancer is acute myeloid leukemia, myelodysplastic syndrome or chronic myeloid leukemia.

13. A preparation method for the CAR-T cell according to any one of claims 1 to 5, comprising: introducing the nucleic acid molecule according to claim 7 or 8 into a T cell for stable expression to obtain the CAR-T cell.

14. A method for the prevention or treatment of a CLL1 target-related disease, comprising: administering the CAR-T cell according to any one of claims 1 to 5 or the pharmaceutical composition according to claim 9 to a subject suffering from the CLL1 target-related disease.

15. The method according to claim 14, wherein the CLL1 target-related disease is CLL1-positive cancer.

16. The method according to claim 15, wherein the CLL1-positive cancer is acute myeloid leukemia, myelodysplastic syndrome or chronic myeloid leukemia.

**Signal   CLL1-VHH-16   Hinge CD8a   4-1BB   CD3ζ**

FIG. 1

FIG. 2

FIG. 3

FIG. 4

FIG. 5

## INTERNATIONAL SEARCH REPORT

| International application No. |
|---|
| **PCT/CN2023/140087** |

**A. CLASSIFICATION OF SUBJECT MATTER**

C12N5/10(2006.01)i; C07K19/00(2006.01)i; C12N15/62(2006.01)i; A61K39/00(2006.01)i; A61P35/02(2006.01)i

According to International Patent Classification (IPC) or to both national classification and IPC

**B. FIELDS SEARCHED**

Minimum documentation searched (classification system followed by classification symbols)

IPC:C12N; C07K; A61K; A61P

Documentation searched other than minimum documentation to the extent that such documents are included in the fields searched

Electronic data base consulted during the international search (name of data base and, where practicable, search terms used)

CJFD, CNTXT, ENTXTC, ENTXT, VENWFB, WFCCP, WFCD, WFCJ, WFCOA, WFCSTA, WFFCP, WFFD, WFFJ, WFFOA, WFS, WFSTR, PubMed, ISI, CNKI, GenBank, STN, Baidu: 单域抗体, CAR, CLL1, CAR-TCLEC12A, C型凝集素样分子1, 白血病, CCML, single domain, 嵌合抗原受体, 4-1BB, CD3ζ, CD8a, 铰链区, 跨膜区, 胞内信号区, SEQ ID NO: 1

**C. DOCUMENTS CONSIDERED TO BE RELEVANT**

| Category* | Citation of document, with indication, where appropriate, of the relevant passages | Relevant to claim No. |
|---|---|---|
| Y | US 2022354890 A1 (LI, G.C.) 10 November 2022 (2022-11-10)<br>claims 1-8 and 15, and description, paragraphs 48-50 | 1-16 |
| Y | CN 115772222 A (CARBIOGENE THERAPEUTICS CO., LTD.) 10 March 2023 (2023-03-10)<br>claims 1-10 | 1-16 |
| Y | CN 116004547 A (CARBIOGENE THERAPEUTICS CO., LTD.) 25 April 2023 (2023-04-25)<br>description, paragraphs 77 and 78 | 2-16 |
| Y | CN 115850505 A (HEYUAN KANGHUA PHARMACEUTICAL TECHNOLOGY (BEIJING) CO., LTD.) 28 March 2023 (2023-03-28)<br>claims 1-10, and description, figure 1 | 1-16 |
| PX | CN 116445415 A (CARBIOGENE THERAPEUTICS CO., LTD.) 18 July 2023 (2023-07-18)<br>entire document | 1-16 |

☑ Further documents are listed in the continuation of Box C.     ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **29 January 2024** | **08 February 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/140087**

| C. | DOCUMENTS CONSIDERED TO BE RELEVANT | |
|---|---|---|
| Category* | Citation of document, with indication, where appropriate, of the relevant passages | Relevant to claim No. |
| PX | CN 116515765 A (CARBIOGENE THERAPEUTICS CO., LTD.) 01 August 2023 (2023-08-01) entire document | 1-16 |

Form PCT/ISA/210 (second sheet) (July 2022)

## INTERNATIONAL SEARCH REPORT

| International application No. |
|---|
| **PCT/CN2023/140087** |

**Box No. I    Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2023/140087** |

| **Box No. II**      **Observations where certain claims were found unsearchable (Continuation of item 2 of first sheet)** |
| --- |

This international search report has not been established in respect of certain claims under Article 17(2)(a) for the following reasons:

1. ☑ Claims Nos.: **14-16**
   because they relate to subject matter not required to be searched by this Authority, namely:

   Claims 14-16 relate to a method for preventing or treating a CLL1 target-related disease, and therefore do not comply with PCT Rule 39.1(iv). The search is performed on the basis of the corresponding pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| International application No. |
| --- |
| **PCT/CN2023/140087** |

| Patent document cited in search report | | | Publication date (day/month/year) | Patent family member(s) | | | Publication date (day/month/year) |
| --- | --- | --- | --- | --- | --- | --- | --- |
| US | 2022354890 | A1 | 10 November 2022 | WO | 2022120942 | A1 | 16 June 2022 |
| | | | | GB | 202207722 | D0 | 13 July 2022 |
| | | | | GB | 2606869 | A | 23 November 2022 |
| | | | | EP | 4039712 | A1 | 10 August 2022 |
| | | | | JP | 2023510465 | A | 14 March 2023 |
| | | | | KR | 20220084040 | A | 21 June 2022 |
| | | | | AU | 2020480789 | A1 | 04 August 2022 |
| CN | 115772222 | A | 10 March 2023 | None | | | |
| CN | 116004547 | A | 25 April 2023 | None | | | |
| CN | 115850505 | A | 28 March 2023 | None | | | |
| CN | 116445415 | A | 18 July 2023 | None | | | |
| CN | 116515765 | A | 01 August 2023 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ENGELS B** ; **CAM H et al.** Retroviral Vectors for High-Level Transgene Expression in T Lymphocytes [J. *Human Gene Therapy*, 2003, vol. 14 (12), 1155-1168